(19) Europäisches Patentamt
European Patent Office
Office européen des brevets

(11) **EP 3 811 896 B1**

(12) **EUROPÄISCHE PATENTSCHRIFT**

(45) Veröffentlichungstag und Bekanntmachung des Hinweises auf die Patenterteilung:
**01.05.2024 Patentblatt 2024/18**

(51) Internationale Patentklassifikation (IPC):
**A61C 8/00** (2006.01) **A61L 27/10** (2006.01)
**C04B 35/486** (2006.01) **A61L 31/02** (2006.01)
**A61L 27/50** (2006.01)

(21) Anmeldenummer: **20202797.5**

(22) Anmeldetag: **20.10.2020**

(52) Gemeinsame Patentklassifikation (CPC):
**A61C 8/0012; A61L 27/10; A61L 27/50;**
**C04B 35/486;** A61C 2008/0046; C04B 2235/3217;
C04B 2235/3224; C04B 2235/3225;
C04B 2235/3229; C04B 2235/765; C04B 2235/963

(54) **DENTALIMPLANTAT**

DENTAL IMPLANT

IMPLANT DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2019 EP 19204545**
**13.12.2019 EP 19216134**

(43) Veröffentlichungstag der Anmeldung:
**28.04.2021 Patentblatt 2021/17**

(73) Patentinhaber:
• **Rohr, Nadja**
**4051 Basel (CH)**
• **Fischer, Jens**
**6622 Ronco sopra Ascona (CH)**

(72) Erfinder:
• **Fischer, Jens**
**8805 Richterswil (CH)**
• **Rohr, Nadja**
**4051 Basel (CH)**
• **Nebe, J. Barbara**
**18095 Rostock (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
EP-A1- 2 545 881        WO-A1-2011/054119
WO-A1-2016/039419        JP-A- 2002 362 972

**Beschreibung**

Die Erfindung betrifft ein Dentalimplantat mit einem keramischen enossalen Bereich zum Einwachsen in einen Kieferknochen, wobei der keramische enossale Bereich Zirkoniumdioxid umfasst und wobei das keramische Zirkoniumdioxid ein Gewichtsverhältnis von tetragonaler zu monokliner Phase von größer 85:15 aufweist sowie ein Verfahren zu dessen Herstellung.

Dentalimplantate sind als Versorgungsvariante für den Ersatz fehlender Zähne und als Verankerungselemente für festsitzende oder abnehmbare prothetische Rekonstruktionen in den verschiedensten Formen und Ausgestaltungen bekannt. Dentalimplantate verfügen üblicherweise über einen enossalen Bereich, der zur Verankerung des Implantats im Knochen dient, und einen Aufbauteil ("Abutment"), der entweder bereits mit dem Implantat verbunden ist ("einteiliges Implantat") oder mit dem enossalen Teil verbunden werden kann ("zweiteiliges Implantat"). Das Abutment dient der Aufnahme der prothetischen Versorgung. Der enossale Teil ist im Allgemeinen als Schraube ausgeführt, um unmittelbar nach dem Einbringen des Implantats eine gute Stabilität ("Primärstabilität") zu erreichen. Im Zuge der Knochenheilung kommt es zu einer Anlagerung des Knochengewebes an das Implantat und damit zu einem dauerhaften Verbund zwischen Knochen und Implantat. Dieser Prozess wird als Osseointegration bezeichnet.

Eine Vielzahl von Materialien wird im Stand der Technik für Dental-Implantate beschrieben. Bekannt sind im Allgemeinen Zahnimplantate aus Metall, beispielsweise Titan. Titan wird als biokompatibel bewertet. Zudem existieren anorganische Werkstoffe, die für Zahnimplantate zum Einsatz kommen. Bewährt haben sich hier insbesondere Keramiken auf Basis von Zirkoniumdioxid (ZrO$_2$). Es ist von der Festigkeit her vergleichbar mit Titan und weist überdies eine zahnähnliche Farbe auf. Klinische Studien belegen eine Überlebensrate von Zirkoniumdioxid-Implantaten von über 98% nach 3 Jahren, was vergleichbar ist mit Titanimplantaten (Balmer M, Spies BC, Vach K, Kohal R-J, Hämmerle CHF, Jung RE. Three-year analysis of zirconia implants used for single-tooth replacement and three-unit fixed dental prostheses: A prospective multicenter study. Clin Oral Implants Res. 2018; 29:290-299).

Zirkoniumdioxid ist eine hochfeste Keramik, deren Festigkeit neben der Stabilität der chemischen Bindung zwischen Zirkonium und Sauerstoff auch durch eine Umwandlungs- oder Transformationsverstärkung erreicht wird. Zirkoniumdioxid liegt in drei verschiedenen Kristallmodifikationen vor. Zwischen der Schmelztemperatur (2680 °C) und 2370 °C tritt die kubische Phase auf, zwischen 2370 °C und 1170 °C die tetragonale und darunter die monokline, die also auch bei Raum- oder Körpertemperatur stabil ist. Die Phasenübergänge sind reversibel, das heißt bei Über- oder Unterschreiten der genannten Temperaturen finden die Phasenübergänge jeweils als Hin- oder Rückreaktion statt. Allerdings kann durch Zugabe von beispielsweise Yttriumoxid die tetragonale Phase bei Raumtemperatur stabilisiert werden. Dieser Zustand ist metastabil. Aktivierungsenergie, beispielsweise durch eine mechanische Spannung, löst den Phasenübergang von der tetragonalen zur monoklinen Struktur auch bei Raumtemperatur aus. Dieser Phasenübergang ist mit einer Volumenvergrößerung von etwa 5 % verbunden. Das vergrößerte Volumen führt zu einer Druckvorspannung und stabilisiert das Material. Auf diese Weise kann ein Risswachstum im Bauteil verhindert oder verlangsamt werden, was die oben angeführte Festigkeitssteigerung im Sinne einer Transformationsverstärkung bewirkt.

Die Formgebung keramischer Zahnimplantate, beispielsweise auf Basis von Zirkoniumdioxid, erfolgt beispielsweise indem mit Binder versetzte Granulatpulver zu Stangen verpresst werden, die maschinell abtragend bearbeitet werden. Ein weiteres Formgebungsverfahren ist der Spritzguss. Die erhaltenen Rohlinge müssen in beiden Fällen noch entbindert und dicht gesintert werden. Um die unvermeidbare Restporosität im dichtgesinterten Material, die dessen mechanische Festigkeit beeinträchtigt, zu reduzieren wird unter hohem Druck und hohen Temperaturen eine weitere Verdichtung durchgeführt. Dieser Prozess wird heiß-isostatisches Pressen ("Hippen") genannt. Weiterhin kann die Formgebung auch durch das abtragende Bearbeiten eines bereits dicht gesinterten und gegebenenfalls auch bereits durch Hippen nachverdichteten Rohlings erfolgen.

Es ist für Titanimplantate allgemein akzeptiert dass ein Implantat im enossalen Teil eine raue Oberfläche aufweisen sollte (Ra ≈ 1.5 μm) um eine rasche und dauerhafte Osseointegration zu erreichen (Wennerberg A, Hallgren C, Johansson C, Danelli S. A histomorphometric evaluation of screw-shaped implants each prepared with two surface roughnesses. Clin Oral Impl Res 1998; 9:11-19). Diese Anforderung wurde im Stand der Technik auch auf keramische Zahnimplantate übertragen.

Die im Stand der Technik für den enossalen Bereich eines Zahnimplantats empfohlenen Rauheiten können durch unterschiedlichste Verfahren erzeugt werden. Beispielsweise können durch Sandstrahlen mit Aluminiumoxid unterschiedlicher Körnung und unterschiedlicher Strahlparameter wie Druck und Abstand unterschiedliche Oberflächenrauheiten erzielt werden.

Die EP 1 450 722 B2 lehrt, dass durch Sandstrahlen mit Korund eine maximale Rauhtiefe von 6,4 μm und eine durchschnittliche Rauhtiefe von 4.7 μm erzielt wird. Die so erzeugten Oberflächen zeichnen sich allerdings durch scharfe Kanten und Furchen aus, die durch die Strahlkörper geschlagen werden. Die Kanten und Furchen sind als Fehlstellen auch potenzielle Rissauslöser. An der Oberfläche von Zirkoniumdioxid-Zahnimplantaten entsteht durch die mechanische Bearbeitung eine hohe Konzentration an monokliner Phase.

Durch Laserbearbeitung kann ebenfalls eine definierte Rauheit erzeugt werden. Die Keramik wird sehr lokal erhitzt, geschmolzen und teilweise verdampft. Das rasche Abkühlen nach der Lasereinwirkung kann lokal Spannungen im Material erzeugen, die wiederum die Festigkeit beeinträchtigen können und bei Zirkoniumdioxid-Keramiken zur Bildung der monoklinen Phase führen.

Eine weitere Möglichkeit der Oberflächenstrukturierung besteht im Aufsintern von beispielsweise Zirkoniumdioxid-Partikeln auf eine keramische Oberfläche, wodurch ebenfalls eine raue Oberfläche erzeugt werden kann. Das Risiko ist in diesem Fall ein ungenügender Verbund zwischen dem Implantatkörper und den aufgesinterten Partikeln.

Mit Flusssäure oder anderen Ätzmitteln kann die Oberfläche angeätzt werden. Dann entsteht eine im Mikrometermaßstab zerklüftete Oberfläche, allerdings bei Zirkoniumdioxid-Keramiken mit einem hohen Anteil monokliner Phase. WO 2008/011948 A1, EP 2 543 333 A2 und EP 2 046 235 B1 lehren, dass durch eine Säurebehandlung an der Oberfläche eine mikroporöse Oberfläche entsteht und zudem aufgrund einer Verarmung von Yttriumoxid eine Anreicherung der monoklinen Phase erfolgt, die zu einer Festigkeitssteigerung beiträgt. Es wird hierbei eine Rauheit von 0,5-20 $\mu$m erzeugt. Bei diesen Verfahren ist jedoch nicht bedacht, dass die Zerklüftung der Oberfläche zu potenziellen Defekten führt, woraus trotz des Vorliegens der monoklinen Phase bei Zirkoniumdioxid-Keramiken eine Festigkeitsminderung für das Zahnimplantat resultieren kann.

Da der chemische Angriff der Flusssäure an der dichten Oberfläche nur sehr langsam erfolgt, wird häufig eine Kombination aus Sandstrahlen und Ätzen verwendet. Das Strahlen bringt Oberflächenspannungen und Defekte ein, so dass der Angriff durch das Ätzmittel leichter erfolgt. An der Oberfläche entsteht bei Zirkoniumdioxid-basierten Zahnimplantaten wiederum ein hoher Anteil monokliner Phase. WO 03/045268 A1 beschreibt eine auf diese Art erzeugte Oberfläche mit einer maximalen Rauhtiefe von 1-20 $\mu$m.

EP 2 545 881 A1 offenbart ein Zahnimplantat auf Basis einer Zirkonkeramik, bei dem der monokline Anteil weniger als 1 Volumenprozent beträgt und dessen mittlere Rauhheit Ra zwischen 1 und 5 $\mu$m liegt.

Die WO 2011/054119 A1 bezieht sich auf ein Keramisches Implantat (1), insbesondere Dentalimplantat, mit einem keramischen, enossalen Oberflächenbereich einer enossalen Partie (6) des Implantats, wobei der keramische enossale Oberflächenbereich mindestens in einem axialen Teilbereich eine erste Region aufweist, in welcher die Oberfläche mit einer Oberflächenmodifikation in Form einer Aufrauhung oder porösen Struktur versehen ist ist, und der keramische enossale Oberflächenbereich in diesem Teilbereich mindestens eine zweite Region aufweist, in der die Oberfläche nicht oder nur in einem geringeren Ausmass mit der genannten Oberflächenmodifikation versehen ist.

Obschon im Stand der Technik raue Oberflächen für den enossalen Bereich von Zahnimplantaten empfohlen werden haben raue Oberflächen auch viele Nachteile. So wird beispielsweise von rauen Titanoberflächen berichtet, dass sich beim Einschrauben des Implantats in den Knochen mikroskopisch kleine Partikel von der Oberfläche lösen, sich im periimplantären Gewebe anreichern und sogar in regionale Lymphknoten transportiert werden. Es ist zu erwarten, dass Gleiches auch bei keramischen Implantaten mit rauer Oberfläche passiert. Weiterhin kann die Oberflächenbearbeitung Defekte an der Implantatoberfläche erzeugen, die zu einer Reduzierung der Festigkeit führen, weshalb kleinere Implantat-Dimensionen, die beispielsweise für den unteren Frontzahnbereich benötigt werden, nicht realisiert werden können. Darüber hinaus ist die Herstellung einer rauen Oberfläche ein zusätzlicher Arbeitsschritt. Wenn darauf verzichtet werden kann, ist die Produktion wirtschaftlicher. Ebenso ist die Herstellung der Oberfläche des enossalen Bereichs unter Verwendung von Flusssäure oder anderen aggressiven Ätzmitteln mit einem nicht unerheblichen Gesundheitsrisiko verbunden.

Im Falle einer periimplantären Entzündung ("Periimplantitis") und dem damit verbundenen Knochenabbau wird die raue enossale Oberfläche freigelegt. Bakterien können sich in die raue Oberfläche einnisten und den Entzündungsprozess damit unbeherrschbar machen. Die Entfernung eines zumindest teilweise osseointegrierten Implantats mit einer rauen Oberfläche benötigt ein sehr hohes Drehmoment. Insbesondere bei keramischen Implantaten sorgt das spröde Material für eine Bruchanfälligkeit. Beim gewaltsamen Herausdrehen des Implantats besteht daher die Gefahr einer Implantat-Fraktur. Heute besteht nur die Möglichkeit, die Implantate durch Umfräsen aus dem Kieferknochen zu entfernen, was zu großen Knochendefekten führt.

JP 2002 362972 A beschreibt Zirkoniumdioxidkeramiken, die für künstliche Gelenke, beispielsweise Hüftgelenke, eingesetzt werden können. Entsprechend weisen die in der JP 2002 362972 A beschriebenen Zirkoniumkeramiken extrem glatte Oberflächen mit einer mittleren Rauhheit Ra von 0,02 $\mu$m oder weniger auf. Der monokline Anteil der Zirkonoxidkeramiken liegt dabei unterhalb von einem Prozent.

Eine bioaktive Beschichtung von Titanimplantaten mit Hydroxylapatit wurde in der Annahme durchgeführt, dass die aus Knochenmineral bestehende Oberfläche zu einer verbesserten Osseointegration führt. Allerdings war der Verbund zwischen Hydroxylapatit und Titan nicht dauerhaft. Hydroxylapatit-Partikel haben sich schon beim Eindrehen des Implantats in den Knochen von der Titanoberfläche abgelöst.

Auch für Zirkoniumdioxid-Implantate wurde eine Beschichtung mit Calcium-Phosphaten beschrieben, beispielsweise in der EP 2 359 873 A1. Auch hier ist der Verbund zwischen Implantatmaterial und Beschichtung eine Schwachstelle.

Um einen festeren Verbund zwischen Zirkoniumdioxid-Implantat und Beschichtung zu erreichen wurde ein Bioglas entwickelt, das einen guten chemischen Verbund zum Zirkoniumdioxid gewährleistet und auch einen angepassten Wärmeausdehnungskoeffizienten aufweist, sodass nach dem Aufbringen des Bioglases, das bei hohen Temperaturen erfolgen muss, während der Abkühlung keine unerwünschten Spannungen im Verbund entstehen. Dieses Verfahren ist in der WO 2015/044401 A2 beschrieben.

Alle vorgestellten Verfahren zur Verbesserung der Osseointegration sind aufwendig und bedeuten einen zusätzlichen Verfahrensschritt.

Überraschend wurde gefunden, dass die im Stand der Technik beschriebenen Probleme durch ein Zahnimplantat mit einem erhöhten Anteil an tetragonaler Zirkoniumdioxidphase im Oberflächenbereich des enossalen Bereichs gelöst werden können. Es hat sich überraschend und unerwartet gezeigt, dass die erfindungsgemäßen Zahnimplantate eine verbesserte Biokompatibilität aufweisen und daher die Osseointegration fördern.

Ein Gegenstand der vorliegenden Erfindung ist ein Zahnimplantat umfassend einen keramischen enossalen Bereich zum Einwachsen in einen Kieferknochen, wobei der keramische enossale Bereich eine Zirkoniumdioxid-Keramik umfasst und das keramische Zirkoniumdioxid ein Gewichtsverhältnis von tetragonaler zu monokliner Phase von größer 85:15 aufweist, dadurch gekennzeichnet, dass die Oberfläche des gesamten enossalen Bereichs eine mittlere Oberflächenrauheit gemäß DIN EN ISO 4287 von 0,03 µm bis 0,4 µm, gemessen gemäß DIN EN ISO 4288, aufweist.

Darüber hinaus wurde überraschend gefunden, dass die im Stand der Technik beschriebenen Probleme durch ein Zahnimplantat mit einer geringen Rauheit des enossalen Bereichs gelöst werden können. Es hat sich gezeigt, dass die erfindungsgemäßen Zahnimplantate eine verbesserte Biokompatibilität aufweisen und daher die Osseointegration fördern.

Ein weiterer Gegenstand der vorliegenden Erfindung ist daher ein Zahnimplantat umfassend einen enossalen Bereich zum Einwachsen in einen Kieferknochen, wobei die Oberfläche des enossalen Bereichs eine mittlere Oberflächenrauheit (Ra gemäß DIN EN ISO 4287) von 0,05 µm bis 0,2 µm, gemessen gemäß DIN EN ISO 4288 aufweist.

Der enossale Bereich oder enossale Teil eines Zahnimplantats ist der Bereich, der zur Verankerung im Kieferknochen dient. Die Oberfläche des enossalen Bereichs verwächst mittels Osseointegration mit dem Kieferknochen. Es hat sich überraschend gezeigt, dass eine geringe mittlere Rauheit die Osteoblastenanlagerung und Biokompatibilität des enossalen Bereichs erheblich verbessert. In einer bevorzugten Ausgestaltung der vorliegenden Erfindung weist die Oberfläche des enossalen Bereichs eine mittlere Oberflächenrauheit gemäß DIN EN ISO 4287

von 0,05 µm bis 0,2 µm, gemessen gemäß DIN EN ISO 4288, auf. Eine mittlere Oberflächenrauhheit im Bereich von 0,03 µm bis 0,4 µm, speziell von 0,04 µm oder 0,05 µm bis 0,35 µm als besonders wirksam herausgestellt. Eine zu geringe mittlere Oberflächenrauhheit kann sich hingegen negativ auf die Osseointegration auswirken.

Das erfindungsgemäße Zahnimplantat besteht aus einem Material, welches biokompatibel und gesundheitlich unbedenklich ist.

In einer bevorzugten Ausführungsform der vorliegenden Erfindung besteht das erfindungsgemäße Zahnimplantat ganz oder teilweise aus dem keramischen Zirkoniumdioxid mit einem Gewichtsverhältnis von tetragonaler zu monokliner Phase von mehr als 85:15.

In einer Ausführungsform der vorliegenden Erfindung besteht zumindest der enossale Bereich ganz oder teilweise aus dem keramischen Zirkoniumdioxid umfassend vorzugsweise ein oder mehrere Oxide der Metalle ausgewählt aus Aluminium, Yttrium, Cer, Hafnium, Calcium und Magnesium.

Speziell bevorzugt umfasst der enossale Bereich des Zahnimplantats eine durch Yttriumoxid stabilisierte Zirkoniumdioxidkeramik.

In einer bevorzugten Ausführungsform der vorliegenden Erfindung besteht der enossale Bereich des Zahnimplantats aus einem homogenen Zirkoniumdioxid.

In einer Ausgestaltung weist der enossale Bereich keine Beschichtung auf. Bevorzugt besteht der enossale Bereich aus einer homogenen durch Yttriumoxid stabilisierten Zirkoniumdioxidkeramik. In einer besonders bevorzugten Ausführungsform besteht das erfindungsgemäße Zahnimplantat ganz oder teilweise aus stabilisiertem Zirkoniumdioxid. In einer besonders bevorzugten Ausführungsform wird als Material zur Herstellung des erfindungsgemäßen Zahnimplantats mit Yttriumoxid stabilisiertes Zirkoniumdioxid verwendet, wobei der Anteil an Yttiumoxid-stabilisiertem Zirkoniumdioxid vorzugsweise mindestens 90 Gew.-%, besonders bevorzugt mindestens 95 Gew.-% und insbesondere mindestens 98 Gew.-% beträgt, jeweils bezogen auf das Gesamtgewicht des Zahnimplantats. Durch einen nachträglichen isostatischen Verdichtungsprozess wird die Materialqualität verbessert und das Versagensrisiko des Implantats gesenkt.

Das Material des erfindungsgemäßen Zahnimplantats ist vorzugsweise zahnfarben. Auf diese Weise kann die Grundlage für eine hohe Ästhetik der prothetischen Versorgung gelegt werden und ein "Durchschimmern" des Implantats durch die Schleimhaut wird verhindert.

Es hat sich überraschend gezeigt, dass der enossale Bereich des Zahnimplantats eine verbesserte Biokompatibilität aufweist, wenn zumindest der enossale Bereich des Zahnimplantats eine Zirkoniumdioxidkeramik umfasst oder daraus besteht, wobei die Zirkoniumdioxidkeramik überwiegend in der tetragonalen Phase vorliegt. Die Ansiedlung von Knochenzellen auf dem enossalen Bereich mit einer Zirkoniumdioxidkeramikoberfläche, wobei die Zirkoniumdioxidkeramik zu wenigstens 85 Gew.-% in der tetragonalen Phase vorliegt, ist deutlich verbessert.

In einer speziellen Ausführungsform weist der enossale Bereich eine Zirkoniumdioxidkeramik, vorzugsweise eine Yttriumoxid stabilisierte Zirkoniumdioxidkeramik, auf, welche an der Oberfläche ein Gewichtsverhältnis von tetragonaler zu monokliner Phase von größer 85:15, vorzugsweise von größer 90:10 aufweist. Speziell bevorzugt ist eine Zirkoniumdioxidkeramik, bei der das Gewichtsverhältnis von tetragonaler zu monokliner Phase von größer 85:15 bis kleiner 99:1, insbesondere von 90:10 bis kleiner 98,5:1,5 ist.

Hinsichtlich der Viabilität hat sich ein tetragonaler Anteil von 85 bis weniger als 99 Gew.-%, insbesondere von 90 bis 98,5 Gew.-%, jeweils bezogen auf das Gesamtgewicht der Zirkoniumdioxidkeramik, als besonders günstig erwiesen.

Die Bestimmung der tetragonalen Phase und der monoklinen Phase in einer Zirkoniumdioxid-Keramik kann mittels Röntgenbeugung mit Cu-Kα-Strahlung und anschließender Rietveld-Auswertung vorgenommen werden.

Es hat sich zudem überraschend gezeigt, dass der Kontaktwinkel (Benetzungswinkel), also der Winkel, den ein Flüssigkeitstropfen auf der Oberfläche zu dieser Oberfläche bildet, einen weiteren positiven Einfluß auf die Viabilität der Osteoblasten haben kann. Es hat sich gezeigt, dass besonders gute Viabilitätswerte erhalten werden können, wenn die Oberfläche des enossalen Bereichs des Zahnimplantats einen Kontaktwinkel für Wasser unterhalb von 90°, vorzugsweise von 40 bis 88°, insbesondere von 60 bis 85° aufweist (gemessen bei 25 °C) zeigt.

Ein weiterer Gegenstand der Erfindung ist daher ein Zahnimplantat umfassend einen enossalen Bereich zum Einwachsen in einen Kieferknochen, wobei die Oberfläche des enossalen Bereichs einen Kontaktwinkel für Wasser unterhalb von 90° aufweist.

Besonders bevorzugt sind erfindungsgemäße Zahnimplantate, die neben zuvor genanntem Kontaktwinkel für Wasser zusätzlich die zuvor genannten Oberflächenrauheiten und/oder den zuvor genannten hohen Anteil von tetragonaler Zirkoniumdioxidphase aufweisen.

Ohne an eine Theorie gebunden zu sein wird vermutet, dass die hohe Benetzbarkeit der Implantatoberfläche des enossalen Bereichs durch eine verbesserte Proteinbindung und eine beschleunigte Differenzierung und Reifung von Osteoblasten zu einer besseren Osseointegration führt. Die Hydrophilie der Oberfläche kann verbessert werden, indem die Anlagerung von Kohlenstoff oder Kohlenstoffverbindungen, die an der Atmosphäre zwangsläufig erfolgt, unterbunden wird, z.B. durch Lagerung der Implantate in einer ionischen Lösung. Die Kohlenstoffkontamination kann weiterhin durch eine Behandlung mit Plasma entfernt werden. Eine Entfernung der Kohlenstoffverunreinigung mittels UV-Bestrahlung unmittelbar vor der Implantation ist eine weitere Alternative.

Insbesondere oxidkeramische Implantate können mittels Alkali-Hydroxid-Behandlung hydrophilisiert werden. Dabei wird die Hydrophilisierung durch eine Modifikation von Metalloxiden an der Oberfläche erreicht.

Es hat sich überraschend gezeigt, dass die Benetzbarkeit der erfindungsgemäßen Implantate in dem gewünschten Kontaktwinkelbereich insbesondere für oxidkeramische Oberflächen, speziell für Zirkoniumdioxid-Oberflächen, durch eine Temperaturbehandlung eingestellt werden kann. Bevorzugt werden die erfindungsgemäßen Zahnimplantate, insbesondere solche, die Zirkoniumdioxid umfassen oder aus solchem bestehen, durch eine Temperaturbehandlung oberhalb von 800 °C für mindestens 10 min hydrophilisiert. Bevorzugt erfolgt die Temperaturbehandlung in einem Temperaturbereich von 1000 bis 1500 °C, insbesondere 1100 bis 1400 °C, speziell im Bereich von 1200 bis 1300 °C. Die Dauer der Temperaturbehandlung liegt bevorzugt oberhalb von 20 min, insbesondere zwischen 30 min und 6 h, speziell zwischen 40 min und 2 h. Beispielsweise wurde die Oberfläche eines Zirkondioxid-basierten Zahnimplantats nach dem Verdichtungsprozess poliert und die mittlere Rauheit (Ra-Wert) auf 0,08 μm eingestellt. Der Kontaktwinkel dieser Probe lag bei 87,4°. Diese Probe wurde einer thermischen Behandlung bei 1250 °C über 1 h unterworfen. Nach der thermischen Behandlung der Probe ergab sich ein praktisch unveränderter mittlerer Rauheitswert (Ra-Wert von 0,09 μm) und ein Kontaktwinkel von 66,5°. Vergleichsweise beträgt der Kontaktwinkel einer gemäß EP 2 170 783 B1 hergestellten Oberfläche mit einem Rauheitswert von Ra = 1,16 μm 90,4°.

Gemäß einer bevorzugten Ausführungsform der vorliegenden Erfindung weist das erfindungsgemäße Zahnimplantat einen enossalen Bereich und ein Abutment zur Aufnahme einer prothetischen Versorgung auf. Vorzugsweise ist zwischen dem enossalen Bereich und dem Abutment ein Implantathals zur Anlagerung von Mukosazellen angeordnet.

In einer weiteren Ausgestaltung der vorliegenden Erfindung ist das Zahnimplantat einstückig mit dauerhaft verbundenem Abutment aufgebaut. Alternativ kann das Zahnimplantat der vorliegenden Erfindung auch mehrstückig aufgebaut sein, vorzugsweise mit lösbarem Abutment.

Um einen optimalen Halt des Implantats im Kieferknochen zu erreichen, hat es sich als vorteilhaft erwiesen, den enossalen Bereich des Implantats, also den Bereich, der in den Kieferknochen eingebracht wird, in Form eines Gewindes auszubilden. Daher ist eine Ausführungsform der vorliegenden Erfindung bevorzugt, in der der enossale Bereich des Zahnimplantats ein Gewinde aufweist. Vorzugsweise handelt es sich bei dem Gewinde um ein selbstschneidendes Gewinde. Die Länge des Gewindes ist vorzugsweise so gewählt, dass eine ausreichende Verankerung im Knochen erzielt wird.

Das erfindungsgemäße Zahnimplantat ist so ausgebildet, dass eine optimale Verankerung im Knochen erreicht wird. Um Knochenverlust durch Druckbelastung, beispielsweise bei einem zu tief im Knochen sitzenden Implantat, zu vermeiden, hat es sich als vorteilhaft erwiesen, wenn der maximale Durchmesser des Implantathalses den Durchmesser des enossalen Bereichs des Implantats nicht übersteigt. Daher ist eine Ausführungsform des erfindungsgemäßen Zahnimplantats bevorzugt, in der das Zahnimplantat stiftförmig ausgebildet ist, wobei der maximale Durchmesser des Implantathalses kleiner oder gleich dem maximalen Durchmesser des enossalen Bereichs ist. Auf diese Weise kann ein guter vertikaler Knochenerhalt gewährleistet werden. Der maximale Durchmesser bezeichnet dabei jeweils eine durch die Mittelachse des Zahnimplantats verlaufende Gerade, die den größtmöglichen Abstand zweier Schnittpunkte mit einer das Zahnimplantat im entsprechenden Bereich orthogonal zur Mittelachse umlaufenden, dem Umfang des Zahnimplantats in diesem Bereich entsprechenden Linie bildet. Die Mittelachse des Zahnimplantats entspricht dabei der Achse, die in Richtung der größten Ausdehnung des Implantats durch dessen Mittelpunkt verläuft.

Der Implantathals ist in der Regel der Bereich eines Zahnimplantats, der mit dem Weichgewebe, insbesondere dem Zahnfleisch, in Kontakt kommt und an den sich Weichgewebszellen, wie beispielsweise Mukosazellen und Gingivazellen anlagern sollen, um einen optimalen Halt und eine lange Nutzung des Implantats zu gewährleisten. Um eine Anlagerung der Weichgewebszellen wie Mukosazellen oder Gingivazellen zu fördern, hat es sich als vorteilhaft erwiesen, wenn der Implantathals ebenfalls eine geringe Oberflächenrauheit entsprechend dem enossalen Bereich des Implantats aufweist.

Um die Stabilität des erfindungsgemäßen Zahnimplantats zu verbessern, hat es sich als vorteilhaft erwiesen, wenn das Implantat zwischen dem enossalen Bereich und dem Implantathals, also dort, wo der Übergang von Hartgewebe zu Weichgewebe erfolgt, eine Verjüngung aufweist. Daher ist eine Ausführungsform bevorzugt, in der der Implantathals einen konusförmigen Abschnitt aufweist, bei dem sich vorzugsweise der Durchmesser von der dem Abutment zugewandten Seite zu der dem enossalen Bereich zugewandten Seite verjüngt.

In einer Ausführungsform kann der Implantathals einen konusförmigen Abschnitt und eine den Implantathals orthogonal zur Mittelachse des Zahnimplantats umlaufende Vertiefung, insbesondere eine horizontal umlaufende Rille aufweisen. Es hat sich überraschend gezeigt, dass die Einbeziehung einer solchen Rille den Effekt des Platform-Switching fördert, wobei durch den geringeren Durchmesser im Vergleich zum enossalen Bereich des erfindungsgemäßen Zahnimplantats in diesem Bereich ein guter vertikaler Knochenerhalt beobachtet wurde. Die den Implantathals horizontal umlaufende Rille grenzt vorzugweise direkt an den enossalen Bereich des Implantats an. In einer besonders bevorzugten Ausführungsform ist die Rille zwischen dem enossalen Bereich und dem konusförmigen Abschnitt des Implantathalses angeordnet. In einer besonders bevorzugten Ausführungsform hat diese Rille eine Breite von 0,1 bis 0,4 mm, vorzugsweise 0,15 bis 0,3 mm. Als weiterer Vorteil kann die Rille in dem erfindungsgemäßen Zahnimplantat dazu herangezogen werden, die ideale Einschraubtiefe des Implantats zu kennzeichnen.

In einer bevorzugten Ausführungsform weist der konusförmige Abschnitt des Implantathalses eine Höhe von 0,5 bis 3 mm, vorzugsweise 1,5 bis 2,5 mm auf. Das erfindungsgemäße Zahnimplantat weist weiterhin ein Abutment zur Aufnahme einer prothetischen Versorgung auf. Bei der prothetischen Versorgung handelt es sich beispielsweise um eine Krone oder eine Brücke. Design und Oberfläche des Abutments sind für einen optimalen Verbund des Implantats mit einer prothetischen Versorgung gestaltet. In einer bevorzugten Ausführungsform weist das Abutment daher eine oder mehrere Vertiefungen auf, um einen sicheren Verbund mit der prothetischen Versorgung zu ermöglichen. Eine solche Vertiefung kann beispielsweise dazu verwendet werden, die prothetische Versorgung mittels Klammern oder nach dem Nut-und-Feder-Prinzip zu befestigen.

In einer besonders bevorzugten Ausführungsform weist das Abutment eine Lamellengestaltung auf. Bei diesen Lamellen handelt es sich vorzugsweise um eine oder mehrere horizontale Mikrorillen in der Oberfläche des Abutments. Diese besondere Ausgestaltung der Oberfläche des Abutments verbessert wesentlich die definitive Befestigung der prothetischen Versorgung. Der enossale Bereich kann zudem ein Gewinde aufweisen.

Ein weiterer Gegenstand ist ein Verfahren zur Herstellung eines Zahnimplantats umfassend einen keramischen enossalen Bereich zum Einwachsen in einen Kieferknochen wobei der keramische enossale Bereich Zirkoniumdioxid umfasst und das keramische Zirkoniumdioxid ein Gewichtsverhältnis von tetragonaler zu monokliner Phase von größer 85:15 aufweist, wobei in einem ersten Schritt ein Zahnimplantat bereitgestellt wird, das einen enossalen Bereich mit Zirkoniumdioxid aufweist, und in einem weiteren Schritt eine Erwärmung erfolgt in einem Temperaturbereich und über eine Zeitdauer, die ausreicht um ein Gewichtsverhältnis von tetragonaler zu monokliner Phase von größer 85:15 einzustellen, wobei die Oberfläche des gesamten enossalen Bereichs eine mittlere Oberflächenrauheit gemäß DIN EN ISO 4287 von 0,03 μm bis 0,4 μm, gemessen gemäß DIN EN ISO 4288, aufweist.

Vorzugsweise wird hierzu in einem ersten Schritt ein Zahnimplantat bereitgestellt, das einen enossalen Bereich aufweist, wobei der enossale Bereich, gegebenenfalls durch Anwendung subtraktiver Verfahren, auf eine mittlere Oberflächenrauheit von kleiner 0,5 μm eingestellt wird und anschließend ein Erwärmschritt erfolgt. Bevorzugt erfolgt die Einstellung der mittleren Oberflächenrauheit des enossalen Bereichs durch Polieren. Erfindungsgemäß ist der Bereich der mittleren Oberflächenrauhheit von 0,03 bis 0,4 μm, insbesondere von 0,04 bis 0,35 μm.

Speziell wird ein Bereich von tetragonaler zu monokliner Phase von 0,5:15 bis kleiner 99:1, insbesondere von 90:10 bis 98,5:1,5 eingestellt.

Die Temperaturbehandlung erfolgt bevorzugt oberhalb von 800 °C für mindestens 10 min. Weiter bevorzugt erfolgt die Temperaturbehandlung in einem Temperaturbereich von 1000 bis 1500 °C, insbesondere 1100 bis 1400 °C, speziell im Bereich von 1200 bis 1300 °C. Die Dauer der Temperaturbehandlung liegt bevorzugt oberhalb von 20 min, insbesondere zwischen 30 min und 6 h, speziell zwischen 40 min und 2 h.

Der thermische Prozess hat überdies den Effekt, dass die Oberfläche des Implantates gereinigt wird und das Implantat so direkt und ohne weitere Reinigungsschritte sterilisiert werden kann.

In einer Ausgestaltung des erfindungsgemäßen Verfahrens kann die Bereitstellung des Zahnimplantats durch subtraktive und/oder additive Formgebungsverfahren erfolgen, vorzugsweise durch Pressen, Drucken, Spritzgießen oder Casting.

Beispiele:

Es wurden mehrere Zirkoniumdioxidkeramik-Prüfkörper mit mittleren Oberflächenrauheiten (Ra) und tetragonalem Anteil (T), wie in den Abbildungen bezeichnet, hergestellt und anschließend wie nachfolgend beschrieben hinsichtlich ihrer Eigenschaften für Zahnimplantate untersucht.

Rauheit

Die mittlere Rauheit Ra gemäß DIN EN ISO 4287 wurde mittels Profilometrie (Hommeltester T1000/TKK50, Hommelwerke, Schwenningen, Deutschland) und einem Tastspitzenradius von 5 $\mu$m 90° (Hommel T1E) gemäß DIN EN ISO 4288 bestimmt.

Monokliner Anteil

Der monokline Anteil der Prüfkörper wurde mittels Pulverdiffraktometrie wie folgt bestimmt:

Gerät: D8 Advance, Software DIFFRAC plus XRD Commander, Version 2.6.1, Bruker, Karlsruhe, Deutschland

Quelle: Kupfer Ko-Strahlung, 1,5406 Å: 40 kV, 40 mA, Nickel K$\beta$-Filter

Detektor: Szintillationszähler mit 0,1 mm Spaltweite

Goniometer-Radius: 250 mm, drehende Probe

Messparameter: Bregg-Brentano Geometrie, coupled two theta/theta, Messbereich 22°-65° 2Theta, Schrittweite 0,02°, Zeit pro Schritt 1,0 Sekunden, Messzeit 36 Min.

Auswerte-Software: BRUKER AXS TOPAZ, Version 4.2 Quantitative Phasenanalyse nach der Rietveld-Methode mit TOPAZ-Software, Hintergrund Chebyshev polynomial

pdf-Datenbasis monoklin: PDF 78-0048 $ZrO_2$

pdf-Datenbasis tetragonal: PDF 79-1769 ZrOz

Benetzbarkeit

Prüfkörper mit einem Durchmesser von 13 mm und einer Höhe von 2 mm wurden im Ultraschallbad mit 70% Ethanol, gefolgt von destilliertem Wasser für je 5 min gereinigt und danach im Ofen (FED-240, Binder, Tuttlingen, Deutschland) bei 200 °C für 2 h sterilisiert.

Der Kontaktwinkel von Wasser wurde an 5 Prüfkörpern pro Gruppe mit einem Kontaktwinkelmessgerät (DSA100, Krüss, Hamburg, Deutschland) ermittelt. Fünf Tropfen mit einem Volumen von 0.5 $\mu$l pro Prüfkörper wurden bei Raumtemperatur (25 °C) mittels der Technik des liegenden Tropfens vermessen.

Zellverhalten

Humane Osteoblasten der Zelllinie MG-63 (American Type Culture Collection ATCC, CRL1427) wurden in Dulbecco's modified eagle medium (DMEM + GlutaMAX -I + 4.5 g/l D-Glucose + Pyruvate; gibco, Thermo Fisher Scientific, Waltham, USA) mit 10% fötalem Kälberserum (FBS superior standardized S0615 0879F, Biochrom, Berlin, Germany) und 1% Antibiotikum (gentamicin, ratiopharm, Ulm, Germany) kultiviert. Die Oberflächen der Prüfkörper wurden mit MG-63 Zellen der Passagen 8-23, welche 70-80% Konfluenz aufwiesen besäht und in 24-well Platten bei 37 °C in einer humiden Atmosphäre mit 5% $CO_2$ für die respektiven Zeiten gelagert. Alle Zellexperimente wurden drei Mal wiederholt.

Zellviabilität

Die mitochondriale Dehydrogenaseaktivität wurde mittels MTT assay (3-(4,5-dimethylthiazol-2-yl)-2,5-diphenyltetrazolium bromide) ermittelt um die Viabilität der auf den Prüfkörpern wachsenden Zellen zu testen. Dazu wurde pro Prüfkörper ein Tropfen (120 $\mu$l) Zellmedium mit 5 x 104 Osteoblasten (Zelllinie MG-63) enthält vorsichtig platziert (n=2 pro Gruppe) und für 20 min inkubiert bis sich die Zellen auf den Oberflächen abgesetzt hatten. Darauf wurde 1 ml Zellkulturmedium pro Well hinzugefügt, um die Prüfkörper vollständig mit Medium zu bedecken und die Well-Platte wurde für 24 h inkubiert. Nach 24 h wurden die Prüfkörper in neue Wellplatten überführt und MTS Lösung (CellTiter 96 ONE-Solution Cell Proliferation Assay, Promega, Madison, USA) mit Zellkulturmedium im Verhältnis 1:5 zu jedem Prüfkörper hinzugefügt. Eine Blindkontrolle von jeder Gruppe ohne Zellen (Blank) sowie Zellen, die auf einer Polystyreneplatte gewachsen waren, dienten als Kontrolle. Nach 80 min fand ein ausreichender Farbumschlag statt und die Lösung von den jeweiligen Prüfkörpern wurde in eine 96-well Platte transferiert. Von jedem Prüfkörper wurden 3 x 80 $\mu$l ausgewertet. Die optische Dichte (OD) wurde bei 490 nm mit einem Mikroplattenleser (Anthos, Mikrosysteme, Krefeld, Germany) analysiert. Die relative Zellviabilität wurde mit folgender Formel berechnet:

$$\text{Relative Zellviabilität} = (OD_{\text{Prüfkörper}} - OD_{\text{blank Prüfkörper}})/(OD_{\text{Kontrolle Polystyrene}} - OD_{\text{blank Kontrolle Polystyrene}}).$$

Zellausbreitung

Die Zellausbreitung wurde auf allen Oberflächen nach 20 min sowie nach 24 h ermittelt. Aufgrund der Opazität der Prüfkörper wurden die Osteoblasten vor dem Aussähen von 3 x 104 Zellen pro Prüfkörper mit roter Fluoreszenzfarbe (PKH-26, Sigma-Aldrich, Steinheim, Germany) eingefärbt. Nach 20 min oder 24 h wurden die Zellen zwei Mal gewaschen mit PBS (dulbecco's phosphate buffered saline, Sigma-Aldrich) fixiert mit 4% Paraformaldehyd für 10 min bei Raumtemperatur, erneut mit PBS gewaschen und auf einem Objektträger eingebettet (Fluoroshield with DAPI, Sigma-Aldrich). Die Zellen wurden mit einem Wasserimmersionsobjektiv (C Apochromat 40x, Carl Zeiss, 1.2 W) bei einer Wellenlänge von 546 nm mit einem konfokalen Lasermikroskop (LSM780, Carl Zeiss, Oberkochen, Germany; ZEN 2011 software black version, Carl Zeiss) aufgenommen. Der Mittelwert der Zellausbreitungsfläche in $\mu$m2 von 40 Zellen pro Prüfkörper wurde mit einer Bildanalysesoftware (ImageJ, v2.0.0) ausgewertet.

Figurenbeschreibung:

Abb. 1: Korrelation zwischen Kontaktwinkel und Viabilität für die entsprechenden Oberflächen mit der jeweiligen mittleren Oberflächenrauheit Ra und tetragonalem Anteil (T) in Gew.-%.
Untersuchungen zur Viabilität von humanen Osteoblasten (MG-63) auf diesen Oberflächen zeigen Korrelation der Viabilität mit dem Kontaktwinkel (Abb. 1).

Abb. 2: Korrelation zwischen tetragonalem Anteil und Viabilität

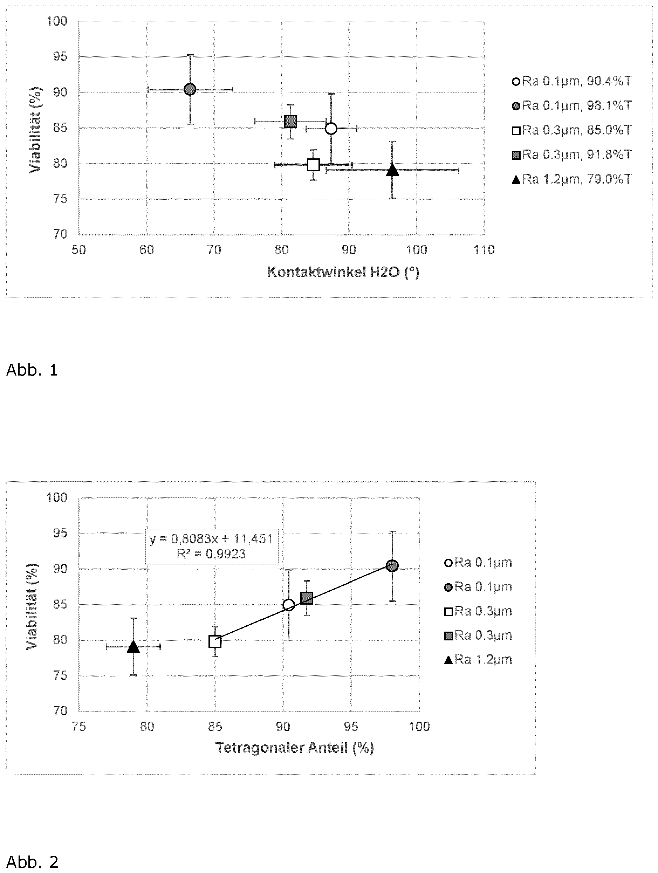

Überraschend hat sich gezeigt, dass eine eindeutige und lineare Korrelation zwischen der Viabilität der Osteoblasten und dem Anteil an tetragonaler Phase an glatten Zirkonoxid-Oberflächen besteht (Abb. 2). Das bedeutet, dass die Viabilität durch die Rückumwandlung der oberflächlich aufgrund des Bearbeitungsprozesses entstehenden monoklinen Phase in die tetragonale Phase wesentlich und vorhersagbar verbessert wird, während die Rauheit keinen signifikanten Einfluss auf die Viabilität hat. Die Oberflächenkonditionierung muss also bei Zirkonoxid-Implantaten nicht nur auf die Verbesserung der Hydrophilie, sondern im Wesentlichen auf die Erzeugung der tetragonalen Phase an der Oberfläche ausgerichtet sein.

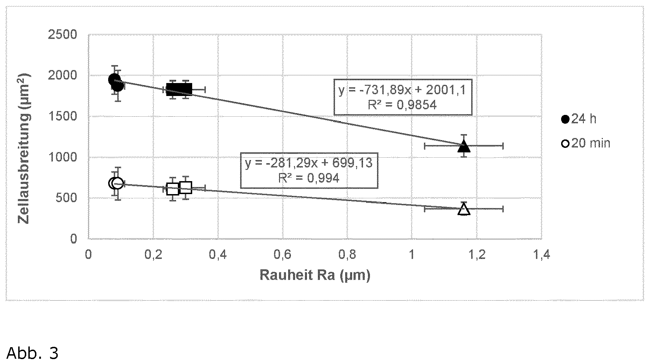

Abb. 3: Korrelation zwischen Rauheit und Zellausbreitung

Weiterhin hat sich überraschend gezeigt, dass die Zellausbreitung der Osteoblasten auf glatten Oberflächen besser ist als auf einer rauen Oberfläche (Abb. 3), was ein zusätzlicher Beleg dafür ist, dass glatte Zirkonoxidoberflächen einen positiven Einfluss auf die Osteoblastenreaktion haben und damit ein hohes osseointegratives Potenzial gegeben ist.

**Patentansprüche**

1. Zahnimplantat umfassend einen keramischen enossalen Bereich zum Einwachsen in einen Kieferknochen, wobei der keramische enossale Bereich eine Zirkoniumdioxid-Keramik umfasst und das keramische Zirkoniumdioxid ein Gewichtsverhältnis von tetragonaler zu monokliner Phase von größer 85:15 aufweist, **dadurch gekennzeichnet, dass** die Oberfläche des gesamten enossalen Bereichs eine mittlere Oberflächenrauheit gemäß DIN EN ISO 4287 von 0,03 $\mu$m bis 0,4 $\mu$m, gemessen gemäß DIN EN ISO 4288, aufweist.

2. Zahnimplantat gemäß Anspruch 1, **dadurch gekennzeichnet, dass** die Oberfläche des enossalen Bereichs eine mittlere Oberflächenrauheit gemäß DIN EN ISO 4287 von 0,05 $\mu$m bis 0,2 $\mu$m, gemessen gemäß DIN EN ISO 4288, aufweist.

3. Zahnimplantat gemäß wenigstens einem der Ansprüche 1 oder 2, **dadurch gekennzeichnet, dass** der enossale Bereich Zirkoniumdioxid-Keramik umfasst, wobei das keramische Zirkoniumdioxid ein Gewichtsverhältnis von tetragonaler zu monokliner Phase von größer 90:10 aufweist.

4. Zahnimplantat gemäß Anspruch 1 bis 3, **dadurch gekennzeichnet, dass** der keramische enossale Bereich zusätzlich ein oder mehrere Oxide der Metalle ausgewählt aus Aluminium, Yttrium, Cer, Hafnium und Erbium, vorzugsweise Yttrium aufweist.

5. Zahnimplantat gemäß einem oder mehreren der Ansprüche 1 bis 4, **dadurch gekennzeichnet, dass** der enossale Bereich des Zahnimplantats eine Zirkoniumdioxid-Keramik, vorzugsweise eine durch Yttriumoxid stabilisierte Zirkoniumdioxid-Keramik, umfasst.

6. Zahnimplantat gemäß einem oder mehreren der Ansprüche 1 bis 5, **dadurch gekennzeichnet, dass** der enossale Bereich aus einer Keramik besteht, die Zirkoniumdioxid und gegebenenfalls Yttriumoxid umfasst, wobei die Summe des Anteils an Zirkoniumdioxid und Yttriumoxid vorzugsweise oberhalb von 80 Gew.-%, besonders bevorzugt oberhalb von 85 Gew.-% und insbesondere oberhalb von 90 oder 92 Gew.-%, speziell oberhalb von 95 Gew.-%, beispielsweise oberhalb von 98 Gew.-%, jeweils bezogen auf das Gesamtgewicht der Keramik, liegt.

7. Zahnimplantat gemäß einem oder mehreren der Ansprüche 1 bis 6, **dadurch gekennzeichnet, dass** das Zahnimplantat einen enossalen Bereich, ein Abutment zur Aufnahme einer prothetischen Versorgung und zwischen Abutment und enossalem Bereich einen Implantathals zur Anlagerung von Gingivazellen aufweist.

8. Zahnimplantat gemäß einem oder mehreren der Ansprüche 1 bis 7 **dadurch gekennzeichnet, dass** das Zahnimplantat einstückig ist oder mehrstückig mit dauerhaft verbundenem oder lösbarem Abutment.

9. Zahnimplantat gemäß einem oder mehreren der Ansprüche 1 bis 8, **dadurch gekennzeichnet, dass** der enossale Bereich ein Gewinde aufweist.

10. Zahnimplantat gemäß einem oder mehreren der Ansprüche 1 bis 9, **dadurch gekennzeichnet, dass** die Oberfläche des enossalen Bereichs einen Kontaktwinkel für Wasser unterhalb von 90°, vorzugsweise von 40 bis 88°, insbesondere von 60 bis 85° aufweist.

11. Verfahren zur Herstellung eines Zahnimplantats umfassend einen keramischen enossalen Bereich zum Einwachsen in einen Kieferknochen wobei der keramische enossale Bereich Zirkoniumdioxid umfasst und das keramische Zirkoniumdioxid ein Gewichtsverhältnis von tetragonaler zu monokliner Phase von größer 85:15 aufweist, wobei in einem ersten Schritt ein Zahnimplantat bereitgestellt wird, das einen enossalen Bereich mit Zirkoniumdioxid aufweist, und in einem weiteren Schritt eine Erwärmung erfolgt in einem Temperaturbereich und über eine Zeitdauer, die ausreicht um ein Gewichtsverhältnis von tetragonaler zu monokliner Phase von größer 85:15 einzustellen, **dadurch gekennzeichnet, dass** die Oberfläche des gesamten enossalen Bereichs eine mittlere Oberflächenrauheit gemäß DIN EN ISO 4287 von 0,03 $\mu$m bis 0,4 $\mu$m, gemessen gemäß DIN EN ISO 4288, aufweist.

12. Verfahren gemäß Anspruch 11, **dadurch gekennzeichnet, dass** der enossale Bereich durch Anwendung subtraktiver Verfahren auf eine mittlere Oberflächenrauheit von 0,05 $\mu$m bis 0,2 $\mu$m, gemessen gemäß DIN EN ISO 4288 eingestellt wird und anschließend der Erwärmschritt erfolgt.

13. Verfahren gemäß Anspruch 12, **dadurch gekennzeichnet, dass** die Erzeugung der mittleren Oberflächenrauheit des enossalen Bereichs durch Polieren erfolgt.

14. Verfahren gemäß Anspruch 11 oder 12 oder 13, **dadurch gekennzeichnet, dass** die Bereitstellung des Zahnimplantats durch subtraktive und/oder additive Formgebungsverfahren erfolgt, vorzugsweise durch Pressen, Drucken, Spritzgießen oder Casting.

15. Verfahren gemäß Anspruch 14, **dadurch gekennzeichnet, dass** die mittlere Rauheit durch den Formgebungsprozess erzeugt wird.

**Claims**

1. A dental implant, comprising a ceramic enossal zone for growing into a jaw, wherein said ceramic enossal zone includes a zirconia ceramic, and the ceramic zirconia has a weight ratio of the tetragonal to the monoclinic phase of more than 85:15, **characterized in that** the surface of the whole enossal zone has a mean surface roughness according to DIN EN ISO 4287 of from 0.03 $\mu$m to 0.4 $\mu$m, as measured according to DIN EN ISO 4288.

2. The dental implant according to claim 1, **characterized in that** the surface of the whole enossal zone has a mean surface roughness according to DIN EN ISO 4287 of from 0.05 $\mu$m to 0.2 $\mu$m, as measured according to DIN EN ISO 4288.

3. The dental implant according to at least one of claims 1 or 2, **characterized in that** said enossal zone comprises a zirconia ceramic, in which the ceramic zirconia has a weight ratio of the tetragonal to the monoclinic phase of more than 90:10.

4. The dental implant according to claim 1 to 3, **characterized in that** said ceramic enossal zone additionally includes one or more oxides of the metals selected from aluminum, yttrium, cerium, hafnium and erbium, preferably yttrium.

5. The dental implant according to one or more of claims 1 to 4, **characterized in that** the enossal zone of the dental implant comprises a zirconia ceramic, preferably an yttria-stabilized zirconia ceramic.

6. The dental implant according to one or more of claims 1 to 5, **characterized in that** the enossal zone consists of a ceramic that includes zirconia and optionally yttria, wherein the total of the proportions of zirconia and yttria is preferably above 80% by weight, more preferably above 85% by weight, and especially above 90% or 92% by weight, specifically above 95% by weight, for example, above 98% by weight, respectively based on the total weight of the ceramic.

7. The dental implant according to one or more of claims 1 to 6, **characterized in that** said dental implant has an enossal zone, an abutment for receiving a prosthetic restoration, and between the abutment and the enossal zone, an implant neck for gingival cell attachment.

8. The dental implant according to one or more of claims 1 to 7, **characterized in that** said dental implant is integral or composite with a permanently connected or detachable abutment.

9. The dental implant according to one or more of claims 1 to 8, **characterized in that** said enossal zone has a thread.

10. The dental implant according to one or more of claims 1 to 9, **characterized in that** the surface of the enossal zone has a contact angle for water of below 90°, preferably from 40° to 88°, especially from 60° to 85°.

11. A process for producing a dental implant, comprising a ceramic enossal zone for growing into a jaw, wherein said ceramic enossal zone includes zirconia, and the ceramic zirconia has a weight ratio of the tetragonal to the monoclinic phase of more than 85:15, wherein, in a first step, a dental implant is provided that has an enossal zone with zirconia, and in another step, heating is performed in a temperature range and over a period sufficient to set a weight ratio of the tetragonal to the monoclinic phase of more than 85:15, **characterized in that** the surface of the whole enossal zone has a mean surface roughness according to DIN EN ISO 4287 of from 0.03 $\mu$m to 0.4 $\mu$m, as measured according to DIN EN ISO 4288.

12. The process according to claim 11, **characterized in that** said enossal zone is set to a mean surface roughness of from 0.05 μm to 0.2 μm, as measured according to DIN EN ISO 4288, by applying subtractive methods, and said heating step is performed thereafter.

**13.** The process according to claim 12, **characterized in that** the achieving of said mean surface roughness of the enossal zone is effected by polishing.

**14.** The process according to claim 11 or 12 or 13, **characterized in that** the providing of the dental implant is effected by subtractive and/or additive shaping methods, preferably by pressing, printing, injection-molding, or casting.

**15.** The process according to claim 14, **characterized in that** said mean roughness is achieved by said shaping method.


**Revendications**

**1.** Implant dentaire, comprenant une zone endo-osseuse céramique à se faire intégrer dans une mâchoire, dans lequel ladite zone endo-osseuse céramique comprend une céramique de zircone, et la zircone céramique présente un rapport pondéral de phases tétragonale à monoclinique supérieur à 85 : 15, **caractérisé en ce que** la surface de la zone endo-osseuse entière présente une rugosité de surface moyenne selon DIN EN ISO 4287 de 0,03 μm à 0,4 μm, mesurée selon DIN EN ISO 4288.

**2.** Implant dentaire selon la revendication 1, **caractérisé en ce que** la surface de la zone endo-osseuse entière présente une rugosité de surface moyenne selon DIN EN ISO 4287 de 0,05 μm à 0,2 μm, mesurée selon DIN EN ISO 4288.

**3.** Implant dentaire selon au moins l'une des revendications 1 ou 2, **caractérisé en ce que** ladite zone endo-osseuse comprend une céramique de zircone, dans lequel la zircone céramique présente un rapport pondéral de phases tétragonale à monoclinique supérieur à 90 : 10.

**4.** Implant dentaire selon les revendications 1 à 3, **caractérisé en ce que** la zone endo-osseuse céramique contient en outre un ou plusieurs oxydes de métaux choisis parmi aluminium, yttrium, cérium, hafnium et erbium, de préférence yttrium.

**5.** Implant dentaire selon l'une ou plusieurs des revendications 1 à 4, **caractérisé en ce que** la zone endo-osseuse de l'implant dentaire comprend une céramique de zircone, de préférence une céramique de zircone stabilisée par l'yttria.

**6.** Implant dentaire selon l'une ou plusieurs des revendications 1 à 5, **caractérisé en ce que** ladite zone endo-osseuse est constituée d'une céramique comprenant de la zircone et éventuellement de l'yttria, où la somme de la proportion de zircone et d'yttria est de préférence supérieure à 80 % en poids, de préférence encore supérieure à 85 % en poids, et notamment supérieure à 90 ou 92 % en poids, de manière spécifique supérieure à 95 % en poids, par exemple, supérieure à 98 % en poids, à chaque fois par rapport au poids total de la céramique.

**7.** Implant dentaire selon l'une ou plusieurs des revendications 1 à 6, **caractérisé en ce que** ledit implant dentaire présente une zone endo-osseuse, un pilier pour recevoir une restauration prothétique, et entre le pilier et la zone endo-osseuse, un col d'implant pour l'accumulation de cellules de gencive.

**8.** Implant dentaire selon l'une ou plusieurs des revendications 1 à 7, **caractérisé en ce que** ledit implant dentaire est intégral ou composite avec un pilier lié de manière permanente ou amovible.

**9.** Implant dentaire selon l'une ou plusieurs des revendications 1 à 8, **caractérisé en ce que** ladite zone endo-osseuse présente un filetage.

**10.** Implant dentaire selon l'une ou plusieurs des revendications 1 à 9, **caractérisé en ce que** la surface de la zone endo-osseuse présente un angle de contact pour l'eau inférieur à 90°, de préférence de 40° à 88°, notamment de 60° à 85°.

**11.** Procédé pour fabriquer un implant dentaire, comprenant une zone endo-osseuse céramique à se faire intégrer dans une mâchoire, dans lequel ladite zone endo-osseuse céramique comprend de la zircone, et la zircone céramique présente un rapport pondéral de phases tétragonale à monoclinique supérieur à 85 : 15, dans lequel, dans une première étape, un implant dentaire ayant une zone endo-osseuse avec de la zircone est procuré, et dans une autre étape, un échauffement s'effectue dans une plage de températures pendant une durée suffisantes pour régler un rapport pondéral de phases tétragonale à monoclinique supérieur à 85 : 15, **caractérisé en ce que** la surface de la zone endo-osseuse entière présente une rugosité de surface moyenne selon DIN EN ISO 4287 de 0,03 $\mu$m à 0,4 $\mu$m, mesurée selon DIN EN ISO 4288.

12. Procédé selon la revendication 11, **caractérisé en ce que** ladite zone endo-osseuse est réglée à une rugosité de surface moyenne de 0,05 $\mu$m à 0,2 $\mu$m, mesurée selon DIN EN ISO 4288, par l'application de méthodes soustractives, et l'étape d'échauffement s'effectue après.

13. Procédé selon la revendication 12, **caractérisé en ce que** la production de la rugosité de surface moyenne de la zone endo-osseuse est effectuée par polissage.

14. Procédé selon la revendication 11 ou 12 ou 13, **caractérisé en ce que** la mise à disposition de l'implant dentaire est effectuée par processus de formation soustractifs et/ou additifs, de préférence par compression, impression, moulage par injection, ou coulée.

15. Procédé selon la revendication 14, **caractérisé en ce que** la rugosité moyenne est produite par le processus de formation.

Abb. 1

Abb. 2

Abb. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1450722 B2
- WO 2008011948 A1
- EP 2543333 A2
- EP 2046235 B1
- WO 03045268 A1
- EP 2545881 A1
- WO 2011054119 A1
- JP 2002362972 A
- EP 2359873 A1
- WO 2015044401 A2
- EP 2170783 B1

**In der Beschreibung aufgeführte Nicht-Patentliteratur**

- **BALMER M ; SPIES BC ; VACH K ; KOHAL R-J ; HÄMMERLE CHF ; JUNG RE.** Three-year analysis of zirconia implants used for single-tooth replacement and three-unit fixed dental prostheses: A prospective multicenter study. *Clin Oral Implants Res.,* 2018, vol. 29, 290-299
- **WENNERBERG A ; HALLGREN C ; JOHANSSON C ; DANELLI S.** A histomorphometric evaluation of screw-shaped implants each prepared with two surface roughnesses. *Clin Oral Impl Res,* 1998, vol. 9, 11-19